# EUROPEAN PATENT APPLICATION

(11) **EP 4 104 814 A1**
(43) Date of publication of application: **21.12.2022**
(21) Application number: 21179508.3
(22) Date of filing: 15.06.2021
(51) Int. Cl.: A61K 8/67, A61K 9/00, A61K 31/714, A61P 31/04, A61Q 17/00

(54) **NOVEL USE OF VITAMIN B12**

(71) Applicant: DSM IP Assets B.V., 6411 TE Heerlen (NL)
(72) Inventor: KOHLER, Lise., 4303 Kaiseraugst (CH); LAURENT, Guillaume Bernard., 4303 Kaiseraugst (CH); MONGIAT, Sebastien., 4303 Kaiseraugst (CH); SFRISO, Riccardo., 4303 Kaiseraugst (CH)
(74) Representative: Berg, Katja

(57) **Abstract**

The present invention relates to the topical use of Vitamin B12 to foster the share of beneficial over non-beneficial skin bacteria.

## Description

The present invention relates to the topical use of Vitamin B12 to foster the share of beneficial over non-beneficial skin bacteria.

Skin is the outermost protective covering of living beings and is the largest organ in the body. One of the main functions of the skin is to form a physical barrier (commonly called the skin barrier) that protects the body from external factors. For example, skin prevents entry of harmful or potentially harmful chemicals and/ or microbes such as e.g. bacteria, fungi and viruses, into the body thereby preventing adverse effects that may be caused thereof.

It is well known that the surface of the skin is colonized by a great variety of microorganisms which form the skin microbiome (often also called skin microbiota). Resident microbiota are found in the upper parts of the epidermis and congregated in and around the hair follicle, as well as in sebaceous and sweat glands.

The understanding of the interaction of cosmetic ingredients and products with skin microbes, has gained more and more importance in the last few years. By now it has been well established that extrinsic factors, such as hygiene practices and/or environmental conditions can lead to a microbial imbalance (also called dysbiosis). In said cases, the skin microbiota can become deranged, with normally dominating, harmless or even beneficial species being underrepresented and normally outcompeted and/ or potentially harmful species increasing to fill the void.

Dysbiosis in the skin microbiome may furthermore contribute to a weakening or even a breakdown of the skin barrier. Such compromised skin then allows for an increased penetration of exogenous substances, such as e.g. bacteria, fungi, polluting agents, irritant agents or allergenic substances, which in turn may cause (further) mild to severe adverse health effects such as skin irritation, allergic reactions or even infections.

Accordingly, microbiome balancing, i.e. fostering and/ or maintaining a healthy balance of the skin microbes to keep the skin in healthy and infection free condition, is often desired by people.

Microbiome balancing may be achieved e.g. by a reduction or elimination of unwanted bacteria on the skin by application of topical compositions comprising one or more antimicrobial compounds. However, most if not all antimicrobial compounds do not work in a selective manner. Thus, said compounds generally do not selectively act only against the unwanted bacteria but often also lead to the removal of the beneficial ones, which in turn may lead to dysbiosis and thus a weakened skin barrier and adverse skin reactions which is not desired.

Microbiome balancing may however also be achieved by way of utilizing compounds which promote the share of desired microbes over undesired ones. Said microbiome balancing is considered as microbiome friendly.

*Staphylococcus epidermidis (S. epidermidis)* is a well-known skin commensal. Current research repeatedly confirms that this bacteria is an important component of a healthy microbiome.

*Staphylococcus hominis (S. hominis)* has been shown to produce hominicin, a bacteriocin with potent activity against clinically relevant strains of *Staphylococcus aureus,* including methicillin-resistant S. aureus (MRSA).

*Staphylococcus aureus (S. aureus)* on the other hand is a Gram positive bacterium which is a pathogenic and redoubtable, particularly due to the emergence of antibioticresistant strains of *S. aureus* such as methicillin-resistant S. aureus (MRSA), leading to a worldwide problem in clinical medicine, e.g. by causing sever skin infections.

Therefore, there is an ongoing need for ingredients which are able to maintain and/ or even foster a healthy skin microbiome by selectively decreasing the share of *S aureus* while increasing the share of *S. epidermidis* and/ or *S. hominis* and which compounds accordingly can be used to prevent and/ or counteract skin dysbiosis.

Surprisingly, it has now been found that Vitamin B12 is suitable as an antimicrobial agent to selectively reduce the growth of S. aureus, in particular in favour of *S. epidermidis* and/ or *S. hominis* in the microbiome of skin.

Said effect can be used to maintain skin healthy, i.e. used in beauty routines for youthful and healthy skin and thus be non-therapeutic, i.e. cosmetic.

Thus, in a first aspect the present invention relates to the use of Vitamin B12 as a selective antimicrobial agent against *S. aureus.*

In a second aspect the present invention relates to a topical non-therapeutic (i.e. cosmetic) use of Vitamin B12 to increase the share *of S. epidermidis* and/ or S. hominis over *S. aureus* in the skin microbiome of an individual in need thereof.

The selective antimicrobial effect as defined herein can also be used for microbiome balancing when applied to the skin.

The above mentioned antimicrobial effect can be used for microbiome balancing when applied on an external surface of the human body.

Thus, the present invention also relates to Vitamin B12 for use in maintaining a healthy skin microbiome by reducing the share of *S. aureus* in favor of the share of one, preferably of both of *S. epidermidis* and *S. hominis,* when applied to the skin.

Said antimicrobial effect can, however, also be used to prevent and/ or treat diseases linked with a dysbiosis of skin microbiota associated with an increased number (over population) of *S. aureus.*

Thus, in another embodiment the present invention also relates to Vitamin B12 for the use in the prevention and/ or treatment of skin dysbiosis in a subject, wherein the skin dysbiosis is associated with an over-colonization by *S. aureus.*

As an overall balanced microbiome leads to the maintenance of an intact skin barrier, the invention also relates to Vitamin B12 for use in counteracting the weakening of the skin barrier caused by a disbalance of the skin microbiome as well as in preventing or reducing the penetration of exogeneous molecules and/or microorganisms following such a weakening such as in particular of *S. aureus.* In particular said disbalance is associated with an increased number of *S. aureus* and/ or a decreased number of at least one, preferably both of *S. epidermidis* and *S. hominis.*

In a particular advantageous embodiment, the present invention relates to a method of reducing the likelihood of a disease linked to and/or caused by a dysbiosis of the skin microbiome which is associated with an increased number of *S. aureus,* preferably combined with a decreased number of at least one, preferably both of *S. epidermidis* and/ or *S. hominis,* in a human being, said method comprising administering a therapeutically effective amount of Vitamin B12 onto a region of an individual's skin and thereby shifting the share in favor of *S. epidermidis* and/ or *S. hominis.*

In another particular advantageous embodiment, the present invention also relates to Vitamin B12 for use in a method of prophylactically or therapeutically treating skin dysbiosis in a subject, wherein said skin dysbiosis is characterized by increased levels of *S. aureus,* preferably combined with a decreased number of at least one, preferably both of *S. epidermidis* and/ or *S. hominis* and wherein said Vitamin B12 selectively increases the share of *S. epidermidis* and / or *S. hominis* over said *S. aureus* in the skin microbiota of a human in need thereof.

As the growth of *S. aureus* is reduced compared to a control not treated with Vitamin B12, the present invention relates in a further advantageous embodiment to a method of suppressing the growth of *S. aureus* in a skin microbiome, the method comprising the step of topically administering a cosmetic or dermatological composition comprising Vitamin B12 to the skin, wherein the level of *S. aureus* in the skin microbiome of the human is lower relative to the level of *S. aureus* in the skin microbiome of a human administered a dermatological or cosmetic composition lacking the Vitamin B12.

In all embodiments of the present invention the selective antimicrobial effect of Vitamin B12 is characterized by reducing the share (and growth) of S. aureus in the skin microbiome, preferably in the presence of at least one, preferably both of *S. epidermidis* and *S. hominis.* In particular concomitantly the share of *S. epidermidis* and/ or *S. hominis* in said skin microbiome is increased, most preferably the share of *S. epidermidis.*

More advantageously, the selective antimicrobial effect of Vitamin B12 is characterized by reducing the growth of *S. aureus, S. epidermidis* and *S. hominis* in the skin microbiome compared to a skin microbiome treated in the absence of Vitamin B12 (optimal growth control), while, however the share of *S. aureus* is over-proportionally decreased and thus the share of *S. epidermidis* and/ or *S. hominis* in said skin microbiome is increased.

In all embodiments of the present invention, the overall population of the microbes in the skin microbiome is reduced compared to a non-treated control, preferably, however, by no more than -100%, most preferably by no more than -75%, such as in particular by no more than -65% after incubation at 37°C for 48h.

Preferably, in all embodiments of the present invention, the share of *S aureus* based on a total share (100%) of *S. aureus* and *S. epidermidis* after treatment is reduced by at least 75 %, preferably by at least 80%, most preferably by 100%.

Preferably, in all embodiments of the present invention, the share of *S aureus,* based on a total share (100%) of *S. aureus* and *S. hominis* after treatment is reduced by at least 10%, preferably by at least 35%, most preferably by 100%.

The term 'preparation' or 'formulation' is used in this document as equivalent to the term 'composition'.

The term 'topical' as used refers to a direct application to skin.

The term microbiome balancing' as used herein refers to maintaining the skin in healthy and infection free condition. Said microbiome balancing is achieved by selectively reducing the share of at least one genus of harmful microbes, preferably *S. aureus* while concomitantly increasing the share of at least one species of beneficial microbes, preferably *S. epidermidis* and/ or *S. hominis.*

The microbiome balancing can be assessed by standard methods in the art as e.g. outlined in the examples.

It is well understood, that a microbiome balancing always takes place in the mutual presence of at least two microbes of a microbiome. Preferably, the microbiome balancing according to the present invention is a balancing of *S. aureus* in the presence of one or both of *S. epidermidis* and/ or *S. hominis.*

The term 'share' as used herein refers to the share of each (individual) microbe with regard to the total number of microbes in % (i.e. with regard to *S. aureus* and *S. epidermidis* or *S. hominis* respectively with regard to *S. aureus, S. epidermidis* and *S. hominis).* Said shares are calculated using the colony count of the respective (individual) microbe divided by the sum of the colony counts of all regarded microbes * 100%.

Any 'count(s)' or colony count(s)' of microbe(s) is given in this document as colony-forming unit (cfu) per milliliter.

The effect according to the present invention is assessable after incubation at 37°C for 48h compared to a control without Vitamin B12 treatment.

The term 'skin' as used in this document, is meant to include the external surface of mammals, especially humans and includes the skin and the scalp. Preferred skin in all embodiments of the present invention is however, facial and body skin such as most preferably facial skin.

The term 'prevention' as used herein refers to lessening the risk of developing a dysbiosis associated with an over-colonization by *S. aureus* and any diseases associated therewith and/or the administration to people in risk of developing a dysbiosis associated with an over-colonization by *S. aureus* and diseases associated therewith.

The term 'treatment' as used herein refers to an amelioration of symptoms, delaying the onset and/ or reduction of the duration of any diseases linked with a dysbiosis associated with an over-colonization by *S. aureus.* The treatment can be prophylactic (cosmetic) or therapeutic.

The exogenous molecules can be in particular irritant substances (hygiene products, solvents, etc.) or allergenic substances (perfumes, house dust, microbial agents).

Vitamin B12 (INCI cyanocobalamin) is e.g. commercially available at DSM nutritional Products Ltd. as Quali^{®}-B.

Preferably, the use level of Vitamin B12 in all embodiments of the present invention is selected in the range of 0.001 wt.-% to 0.1 wt.-%, preferably in the range of 0.001 wt.-% to 0.05 wt.-%, most preferably in the range of 0.001 to 0.025 wt.-%. Further suitable ranges include 0.001 wt.% to 0.01 wt.-%.

In all embodiments of the present invention the microbiome balancing according to the present invention is most preferably characterized by reducing the share of *S. aureus* over the share of *S. epidermidis* and/ or *S. hominis,* preferably in the concomitant presence of *C. acnes.*

Furthermore, as the growth *S. aureus* is decreased by the application of Vitamin B12 compared to control, the present invention also relates to the use of Vitamin B12 as selective antimicrobial agent against *S. aureus,* in particular in the concomitant presence of at least one, preferably all microbes selected from the group consisting of *S. hominis, S. epidermidis* and *C. acnes,* even more in particular to balance the skin microbiome of sebaceous skin and foster skin health.

The term 'use as a selective antimicrobial' as used herein in particular also refers to a method for treating dysbiosis of the skin of a subject, comprising
a) providing an area of skin having a microbiota and a state of dysbiosis
b) providing a topical composition comprising Vitamin B12, and
c) applying said topical composition to the area of skin, and
d) changing the microbiota of the skin by reducing the number of microorganisms promoting dysbiosis, such as in particular *S. aureus* to a higher extend than the number of organisms promoting eubiosis such as in particular *S. epidermidis* and/ or *S. hominis* and changing the state of dysbiosis to a state of eubiosis.

It is important that the balancing of microbial growth allows the skin to remain in a healthy state. Hence, the Vitamin B12 or a composition comprising Vitamin B12, respectively, is in one advantageous embodiment applied on healthy skin and maintains or improves the aesthetic aspect of said skin; in said embodiments of the present invention, preferably, all uses and methods are cosmetic and non-therapeutic.

Thus, a preferred aspect of the invention relates to a non-therapeutical, cosmetic method of maintaining a healthy skin microbiome, said method comprising the step of topically applying a cosmetic composition comprising Vitamin B12 to a subject and optionally appreciating the effect. It is well understood that all definitions and preferences as outlined herein also apply to the method.

The Vitamin B12 or a composition comprising Vitamin B12 can be used to reestablish microbiome balance in patients suffering from a dysbiosis connected with an over colonialization of *S. aureus.*

In all embodiments of the present invention, the Vitamin B12 is preferably applied to the skin incorporated into a cosmetic or dermatological composition.

The amount of the Vitamin B12 in the cosmetic or dermatological composition is preferably selected in the range from 0.001 to 0.5 wt.-%, preferably 0.001 to 0.1 wt.-%, more preferably 0.001 to 0.05 wt.-%, based on the total weight of the composition. Further suitable ranges encompass 0.001 to 0.25 wt.-% or 0.001 to 0.2 wt.-%, based on the total weight of the cosmetic composition.

The term 'cosmetic composition' as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The cosmetic or dermatological compositions according to the present invention are in particular compositions intended to be topically applied to mammalian keratinous tissue such as in particular to human skin or the human scalp. Preferred in all embodiments of the present invention the cosmetic or dermatological compositions are topical cosmetic or dermatological compositions.

The cosmetic or dermatological composition may be a leave-on or a rinse off cosmetic composition, and includes any product applied to a human body primarily for improving appearance, cleansing, odor control or general aesthetics. Preferably the cosmetic or dermatological composition of the present invention are leave-on compositions.

The cosmetic or dermatological compositions according to the present invention preferably further comprise a physiologically acceptable medium, that is to say a medium compatible with keratinous substances, such as the skin, mucosa, and keratinous fibers. In particular the physiologically acceptable medium is a cosmetically respectively dermatologically acceptable carrier.

The term cosmetically respectively dermatologically acceptable carrier refers to all carriers and/or excipients and/or diluents conventionally used in cosmetic compositions or dermatological or pharmaceutical compositions.

The cosmetic or dermatological composition may comprise further ingredients. Such ingredients are particularly surfactants, emulsifiers, thickeners, and oils. Such suitable surfactants, emulsifiers, thickeners, and oils are well known to a person skilled in the art.

Preferably, the cosmetic or dermatological compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

The cosmetic or dermatological compositions in accordance with the invention can be in the form of a liquid, lotion, a thickened lotion, a gel, a cream, a milk, an ointment or a paste.

The cosmetic or dermatological compositions according to the invention generally have a pH in the range from 3-10, preferably in the range from pH of 3-8, most preferred in the range from pH 3.5-7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

The cosmetic or dermatological compositions according to the present invention are in particular skin care preparations, functional preparations and/or hair care preparations such as most in particularly skin or hair care preparations.

Examples of skin care preparations are, in particular, light protective preparations (sun care preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples of hair care preparations which are suitable according to the invention and which may be mentioned are shampoos, hair conditioners (also referred to as hair rinses), hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

In a preferred embodiment, the cosmetic or dermatological compositions according to the present invention are emulsions and/or gels. Even more preferably, the cosmetic or dermatological compositions are emulsions which contain an oily phase and an aqueous phase such as in particular O/W, W/O, Si/W, W/Si, O/W/O, W/O/W multiple or a pickering emulsions.

The amount of the oily phase (i.e. the phase containing all oils and fats including the polar oils) present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the cosmetic or dermatological composition.

The amount of the aqueous phase present in such emulsions is preferably at least 20 wt.-%, such as in the range from 20 to 90 wt.-%, preferably in the range from 30 to 80 wt.-%, most preferably in the range from 30 to 70 wt.-%, based on the total weight of the cosmetic or dermatological composition.

Particularly important is that the Vitamin B12 may have a particular beneficial effect on the skin microbiome, when combined with further skin active ingredients.

Particularly preferred skin active ingredients according to the present invention are plant-derived carbohydrate compounds such as monosaccharides, disaccharides, or polysaccharides, preferably fructans and galactans, as well as saccharide isomerates and polyols, particularly glycerol and 1,3-propanediol, sugar alcohols e.g. maltitol, sorbitol, xylitol, erythritol and isomalt, vitamins such as niacinamide and peptides, such as tripeptides such as in particular SYN^{®}-HYCAN from DSM Nutritional Products Ltd as well as mixtures thereof.

Saccharide isomerate is for example commercially available under trade name Pentavitin^{®} from DSM Nutritional Products AG, Switzerland.

Niacinamide is for example commercially available under trade name Niacinamide PC from DSM Nutritional Products AG, Switzerland.

Examples of monosaccharide include glucose, fructose, galactose and mixtures thereof.

Examples of disaccharides include sucrose, maltose, lactose and mixtures thereof.

Fructans are a category of carbohydrate consisting of fructooligosaccharides (FOS) and inulins, while galactans consist of galactooligosaccharides (GOS). Further preferred prebiotics are resistant starch, pectin, beta-glucans, and xylooligosaccharides.

Further suitable ingredients to be combined with the hyaluronic acid of the present invention in the uses and methods according to the present invention is niacinamide.

Hence, it is preferred that a composition comprising the Vitamin B12 and at least one skin active ingredient, preferably saccharide isomerate and/or niacinamide, as described above are used to balance the microbiome of human skin and/or hair, particularly of the scalp or the scalp hair, with all the definitions and preferences as given herein.

The following examples are provided to further illustrate the compositions and effects of the present invention. These examples are illustrative only and are not intended to limit the scope of the invention in any way.

### Experimental set-up:

Assay: The following strains were used to assess the effect of vitamin B12 on their growth behavior.

| **Microbial strains** | **ATCC reference** |
|---|---|
| *Staphylococcus aureus* | 6538 |
| *Staphylococcus epidermidis* | 12228 |
| *Staphylococcus hominis* | 27844 |

Culture conditions, adapted to the correct growth of all the strains together in a co-culture, were established previously. In aero/anaerobic conditions and in the same culture medium, the strains coexist as equilibrium for 48 hours.

The activity of vitamin B12 was studied as follows:
- Samples (0.1 wt.-%, 0.01 wt.-%, or 0.001 wt.-% of the vitamin B12) were deposited in a microplate culture medium at different concentrations;
- Samples and control (optimal growth medium) were contaminated by mixing the strains, whose initial proportion allows the population to be approximately at 10⁶ CFU/mL after 3 to 6 h;
- An aliquot of culture was picked up at 0 and at 48 hours of incubation;
- The aliquot was deposited on specific agar plate for each strain and incubated for 48 hours at 37 °C in aerobic/anaerobic conditions.
- After incubation, each strain was counted. For each sample, the analysis was performed in triplicate. The results are presented below.
Inoculum concentration: 1E+08 CFU/mL

Table 1 shows the counts of colony (CFU/mL) and shares of the respective microbes after at 48 hours of incubation at 37°C.

**Table 1: Share S. aureus/S. epidermidis**

| **Microbe** | Growth medium control | | 0.001% Vitamin B12 | | 0.01% Vitamin B12 | | 0.1% Vitamin B12 | |
|---|---|---|---|---|---|---|---|---|
| | **CFU/mL** | **Share** | **CFU/mL** | **Share** | **CFU/mL** | **Share** | **CFU/mL** | **Share** |
| *S.aureus* | 1.55E+09 | 51% | 1.25E+08 | 9% | 5.00E+07 | 8% | 0.00E+00 | 0% |
| *S.epidermidis* | 1.50E+09 | 49% | 1.25E+09 | 91% | 5.75E+08 | 92% | 1.15E+09 | 100% |
| **Total** | 3.05E+09 | 100% | 1.38E+09 | 100% | 6.25E+08 | 100% | 1.15E+09 | 100% |

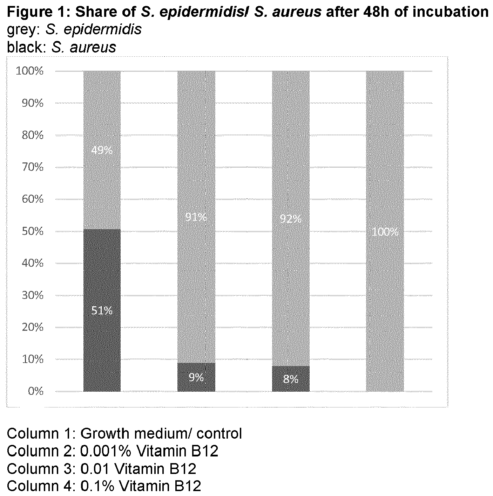

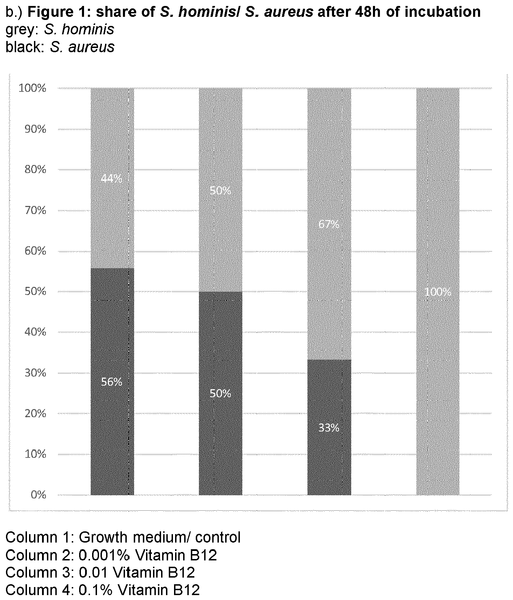

Figure 1 illustrates the share of the respective microbe after 48 hours of incubation at 37°C by means stacked bar chart illustrating the prebiotic effect of the vitamin B12 according to the present invention reflected by an increase in the share in *S. epidermidis* and a reduction in the share of *S. aureus.*

Table 2 shows the counts of colony (CFU/ml) and shares of the respective microbes after at 48 hours of incubation at 37°C.

**Table 2: Share S. aureus/S. hominis**

| **Microbe** | Growth medium control | | 0.001% Vitamin B12 | | 0.01% Vitamin B12 | | 0.1% Vitamin B12 | |
|---|---|---|---|---|---|---|---|---|
| | **CFU/mL** | **Share** | **CFU/mL** | **Share** | **CFU/mL** | **Share** | **CFU/mL** | **Share** |
| *S.aureus* | 1.55E+09 | 56% | 1.25E+08 | 50% | 5.00E+07 | 33% | 0.00E+00 | 0% |
| *S.hominis* | 1.23E+09 | 44% | 1.25E+08 | 50% | 1.00E+08 | 67% | 5.00E+07 | 100% |
| **Total** | 2.78E+09 | 100% | 2.50E+08 | 100% | 1.50E+08 | 100% | 5.00E+07 | 100% |

Figure 2 illustrates the share of the respective microbe after 48 hours of incubation at 37°C by means stacked bar chart illustrating the selective antimicrobial effect of Vitamin B12 reflected by an increase in the share in *S. hominis* and a reduction in the share of *S. aureus.*

### Formulation examples

**1. Aqueous Serum**

| **Phase** | **ingredients** | **INCI Name** | **% w / w** |
|---|---|---|---|
| A | Water Dem | AQUA | Ad 100 |
| | SODIUM GLUCONATE GRANULAR | SODIUM GLUCONATE | 0,20 |
| | Dermosoft^{®} Octiol | CAPRYLYL GLYCOL | 0,20 |
| | Eumulgin CO 60 | PEG-60 HYDROGENATED CASTOR OIL | 0,30 |
| | | | |
| B | **TILAMAR^{®} PDO with NØØVISTA**^{™} | PROPANEDIOL | 3,00 |
| | Cosphaderm Pentiol natural | PENTYLENE GLYCOL | 3,00 |
| | **SYN^{®}-HYCAN** | GLYCERIN, AQUA, MAGNESIUM CHLORIDE, TETRADECYL AMINOBUTYROYLVALYLAMINOBUTYRIC UREA TRIFLUOROACETATE | 2,50 |
| | **ALPAFLOR^{®} ARTEMISIA AO** | ARTEMISIA UMBELLIFORMIS FLOWER EXTRACT, GLYCERIN, AQUA, CITRIC ACID, SODIUM BENZOATE, POTASSIUM SORBATE | 1,00 |
| | **ALPAFLOR^{®} LINUM AO** | GLYCERIN, AQUA, CITRIC ACID, POTASSIUM SORBATE, LINUM ALPINUM FLOWER/LEAF/STEM EXTRACT | 1,00 |
| | | | |
| C | **HYA-ACT^{™} S** | SODIUM HYALURONATE | 0,50 |
| | | | |
| D | **Vitamin B12** | CYANOCOBALAMIN | 0,001 - 0,5 |
| | Water Dem | AQUA | 0,50 |
| | | | |
| E | Citric Acid 10% Solution | CITRIC ACID, AQUA | q.s. |

**2. Cream gel**

| **Phase** | **Ingredients** | **INCI Name** | **% w / w.** |
|---|---|---|---|
| A | WATER DEM. | AQUA | Ad 100 |
| | Edeta BD | DISODIUM EDTA, AQUA | 0,02 |
| | Carbopol 980 | CARBOMER | 0,60 |
| | Blanose CMC 7HF PH | CELLULOSE GUM | 0,15 |
| | Glycerin 86.5% | GLYCERIN | 3,00 |
| | Viscarin PC 209 | CARRAGEENAN | 0,25 |
| | Dermosoft^{®} Octiol | CAPRYLYL GLYCOL | 0,20 |
| | Pentylene Glycol | PENTYLENE GLYCOL | 3,00 |
| | | | |
| B | **HYA-ACT^{™} M** | SODIUM HYALURONATE | 0,20 |
| | **SYN^{®}-AKE** | GLYCERIN, AQUA, DIPEPTIDE DIAMINOBUTYROYL BENZYLAMIDE DIACETATE | 4,00 |
| | **PEPHA^{®}-AGE** | SCENEDESMUS RUBESCENS EXTRACT, AQUA, PHENOXYETHANOL | 1,00 |
| | | | |
| C | Ethanol abs. | ALCOHOL | 3,00 |
| | Perfume Sail Away 49454695 | PARFUM | 0,10 |
| | **VALVANCE^{®} *Look* 100** | SILICA, TITANIUM DIOXIDE | 2,00 |
| | Timiron^{®} Halo White | TITANIUM DIOXIDE, TIN OXIDE | 1,10 |
| | Gemtone Tan Opal G005 | MICA, IRON OXIDES, TITANIUM DIOXIDE | 0,20 |
| | **Vitamin B12** | CYANOCOBALAMIN | 0,001 - 0,5 |
| | | | |
| D | Sodium Hydroxide 30% Solution | AQUA, SODIUM HYDROXIDE | q.s. |

**3. Day cream SPF 30**

| **Phase** | **Ingredients** | **INCI Name** | **% w** / **w** |
|---|---|---|---|
| A | **PARSOL^{®} 1789** | BUTYL METHOXYDIBENZOYLMETHANE | 3,00 |
| | **PARSOL^{®} 340** | OCTOCRYLENE | 8,00 |
| | **PARSOL^{®} EHS** | ETHYLHEXYL SALICYLATE | 5,00 |
| | **PARSOL^{®} SLX** | POLYSILICONE-15 | 1,50 |
| | **PARSOL^{®} Shield** | BIS-ETHYLHEXYLOXYPHENOL METHOXYPHENYL TRIAZINE | 1,50 |
| | Dermofeel^{®} BGC | BUTYLENE GLYCOL DICAPRYLATE/DICAPRATE | 2,00 |
| | Montanov 202 | ARACHIDYL ALCOHOL, BEHENYL ALCOHOL, ARACHIDYL GLUCOSIDE | 1,60 |
| | MONTANOX 60 DF | POLYSORBATE 60 | 0,40 |
| | Cetiol Ultimate | UNDECANE, TRIDECANE | 2,00 |
| | **AMPHISOL^{®} K** | POTASSIUM CETYL PHOSPHATE | 0,30 |
| | Sepiplus 400 | POLYACRYLATE-13, POLYISOBUTENE, POLYSORBATE 20 | 0,50 |
| | | | |
| B | WATER DEM. | AQUA | Ad 100 |
| | Edeta BD | DISODIUM EDTA | 0,05 |
| | **PARSOL^{®} HS** | PHENYLBENZIMIDAZOLE SULFONIC ACID | 2,00 |
| | Tris(hydroxymethyl)aminomethane | TROMETHAMINE | 1,00 |
| | **TILAMAR^{®} PDO with NØØVISTA^{™}** | PROPANEDIOL | 2,00 |
| | Keltrol CG-T | XANTHAN GUM | 0,10 |
| | Pentylene Glycol | PENTYLENE GLYCOL | 3,00 |
| | Dermosoft^{®} Octiol | CAPRYLYL GLYCOL | 0,20 |
| | | | |
| C | **Vitamin B12** | CYANOCOBALAMIN | 0,001 - 0,5 |
| | **ALPAFLOR^{®} GIGAWHITE CB** | MALVA SYLVESTRIS FLOWER/ LEAF/STEM EXTRACT, MENTHA PIPERITA LEAF EXTRACT, PRIMULA VERIS FLOWER EXTRACT, MELISSA OFFICINALIS LEAF EXTRACT, ACHILLEA MILLEFOLIUM FLOWER/LEAF/STEM EXTRACT, GLYCERIN, AQUA, ALCHEMILLAVULGARIS FLOWER/LEAF/STEM EXTRACT, VERONICA OFFICINALIS FLOWER/LEAF/STEM EXTRACT, CITRIC ACID | 2,00 |
| | **ALPAFLOR^{®} EDELWEISS CB** | LEONTOPODIUM ALPINUM FLOWER/LEAF EXTRACT, GLYCERIN, AQUA, CITRIC ACID | 0,50 |
| | **NIACINAMIDE PC** | NIACINAMIDE | 1,00 |
| | Ethanol abs. | ALCOHOL | 6,00 |
| | Perfume Sail Away 49454695 | PARFUM | 0,15 |

**4. Moisturizing day cream**

| **Phase** | **Ingredients** | **INCI Name** | **% w / w** |
|---|---|---|---|
| A | Estol 3650 | GLYCERYL MYRISTATE | 1.50 |
| | Lanette 16 | CETYL ALCOHOL | 1.50 |
| | Finsolv TN | C12-15 ALKYL BENZOATE | 4.00 |
| | Lanol 99 | ISONONYL ISONONANOATE | 2.00 |
| | Brij 72 | STEARETH-2 | 1.50 |
| | Brij 721 | STEARETH-21 | 1.50 |
| | | | |
| B | WATER DEM. | AQUA | Ad 100 |
| | 1,3-Butylenglycol | BUTYLENE GLYCOL | 2.00 |
| | Glycerin 86.5% | GLYCERIN | 3.00 |
| | Edeta BD | DISODIUM EDTA | 0.10 |
| | Keltrol CG-T | XANTHAN GUM | 0.30 |
| | Carbopol Ultrez 21 | ACRYLATES/C10-30 ALKYL ACRYLATE CROSSPOL YMER | 0.25 |
| | Preservative | | q.s. |
| | | | |
| C | WATER DEM. | AQUA | 10.00 |
| | **STAY-C^{®} 50** | SODIUM ASCORBYL PHOSPHATE | 3.00 |
| | Natriumdisulfit | SODIUM METABISULFITE | 0.05 |
| | | | |
| D | **Vitamin B12** | CYANOCOBALAMIN | 0,001 - 0,5 |
| | | | |
| E | Citric Acid Anhydrous | CITRIC ACID | q.s. |

**5. Facial mask scrub**

| **Phase** | **Ingredients** | **INCI Name** | **% w / w** |
|---|---|---|---|
| A | Water Dem | AQUA | Ad 100 |
| | PUROX S | SODIUM BENZOATE | 0,25 |
| | SODIUM GLUCONATE GRANULAR | SODIUM GLUCONATE | 0,20 |
| | Dermosoft^{®} GMCY MB | GLYCERYL CAPRYLATE | 0,30 |
| | Glycerine CremerGLYC 86.5% | GLYCERIN | 3,00 |
| | Cosphaderm Pentiol natural | PENTYLENE GLYCOL | 3,00 |
| | AMIGUM | SCLEROTIUM GUM | 1,50 |
| | | | |
| B | Oramix NS 10 | DECYL GLUCOSIDE | 1,00 |
| | Amisoft CS-22 | SODIUM COCOYL GLUTAMATE, DISODIUM COCOYL GLUTAMATE, AQUA | 2,00 |
| | | | |
| C | Cutina FS 45 | STEARIC ACID, PALMITIC ACID | 8,00 |
| | Lanette 16 | CETYL ALCOHOL | 1,00 |
| | | | |
| D | **VALVANCE^{®} *Touch* 210** | SILICA | 3,00 |
| | Unipure White LC 981 | CI 77891 | 2,00 |
| | | | |
| E | Perfume | PARFUM | 0,10 |
| | **ALPAFLOR^{®} ALP-SEBUM CB** | GLYCERIN, AQUA, EPILOBIUM FLEISCHERI FLOWER / LEAF / STEM EXTRACT, CITRIC ACID | 3,00 |
| | **Vitamin B12** | CYANOCOBALAMIN | 0,001 - 0,5 |
| | | | |
| F | Argan BioEXFOLIATOR 500 | ARGANIA SPINOSA SHELL POWDER | 2,00 |
| | Beige Montmorillonite Clay | MONTMORILLONITE | 15,00 |

**6. Mousse cleanser**

| **Phase** | **ingredients** | **INCI Name** | **% w/w** |
|---|---|---|---|
| A | WATER DEM. | AQUA | Ad 100 |
| | | | |
| B | Oramix NS 10 | DECYL GLUCOSIDE, AQUA | 4,00 |
| | Plantapon LGC Sorb | AQUA, SODIUM LAURYL GLUCOSE CARBOXYLATE, LAURYL GLUCOSIDE | 20,00 |
| | Plantacare 818 UP | COCO-GLUCOSIDE, AQUA | 10,00 |
| | Pentylene Glycol | PENTYLENE GLYCOL | 2,00 |
| | Dermosoft^{®} Octiol | CAPRYLYL GLYCOL | 0,20 |
| | | | |
| C | **PENTAVITIN^{®}** | SACCHARIDE ISOMERATE, AQUA, CITRIC ACID, SODIUM CITRATE | 0,50 |
| | **ALPAFLOR^{®} SCUTELLARIA CB** | GLYCERIN, AQUA, CITRIC ACID, SCUTELLARIA ALPINA FLOWER/LEAF/STEM EXTRACT | 3,00 |
| | **Vitamin B12** | CYANOCOBALAMIN | 0,001 - 0,5 |
| | | | |
| D | Citric Acid 10% Solution | CITRIC ACID, AQUA | q.s. |

**7. Oil cleanser**

| **Phase** | **Ingredients** | **INCI Name** | **% w/w** |
|---|---|---|---|
| A | Sucragel AP V2 | GLYCERIN, AQUA, SUCROSE LAURATE | 20,00 |
| | Sucrablend SP V2 | SUCROSE STEARATE, SUCROSE PALMITATE | 0,50 |
| | CremerGlyc Refined Glycerine Organic | GLYCERIN, AQUA | 9,00 |
| | **ALPAFLOR^{®} SCUTELLARIA CB** | GLYCERIN, AQUA, CITRIC ACID, SCUTELLARIA ALPINA FLOWER/LEAF/STEM EXTRACT | 0,50 |
| | **ALPAFLOR^{®} EDELWEISS CB** | LEONTOPODIUM ALPINUM FLOWER/LEAF EXTRACT, GLYCERIN, AQUA, CITRIC ACID | 0,50 |
| | **Vitamin B12** | CYANOCOBALAMIN | 0,001 - 0,5 |
| | | | |
| B | **ARGAN OIL** | ARGANIA SPINOSA KERNEL OIL | 2,00 |
| | Myritol 318 | CAPRYLIC/CAPRIC TRIGLYCERIDE | Ad 100 |
| | Cetiol C5 | COCO-CAPRYLATE | 5,00 |
| | | | |
| C | Eutanol G | OCTYLDODECANOL | 30,00 |
| | **BEAUACTIVE^{®}** | HYDROXYSTEARIC ACID | 1,00 |

**8. Shower gel**

| **Phase** | **Ingredients** | **INCI Name** | **% w / w** |
|---|---|---|---|
| A | WATER DEM. | AQUA | Ad 100 |
| | Texapon NSO - BZ | SODIUM LAURETH SULFATE, AQUA | 40,00 |
| | Tego Betain F50 | COCAMIDOPROPYL BETAINE, AQUA, SODIUM CHLORIDE | 7,00 |
| | | | |
| B | Antil 171 | PEG-18 GLYCERYL OLEATE/COCOATE, AQUA | 1,70 |
| | | | |
| C | PUROX S | SODIUM BENZOATE | 0,50 |
| | **PENTAVITIN^{®}** | SACCHARIDE ISOMERATE, AQUA, CITRIC ACID, SODIUM CITRATE | 0,50 |
| | **Vitamin B12** | CYANOCOBALAMIN | 0,001 - 0,5 |
| | Citric Acid Anhydrous | CITRIC ACID | q.s |
| | Sodium Chloride | SODIUM CHLORIDE | 0,30 |

**9. Hand Cream**

| **Phase** | **ingredients** | **INCI Name** | **%w/w** |
|---|---|---|---|
| A | Water Dem | AQUA | Ad 100 |
| | SODIUM GLUCONATE GRANULAR | SODIUM GLUCONATE | 0,20 |
| | Dermosoft^{®} Hexiol | 1,2-HEXANEDIOL | 2,00 |
| | Dermosoft^{®} Octiol | CAPRYLYL GLYCOL | 0,20 |
| | **TILAMAR^{®} PDO with NØØVISTA^{™}** | PROPANEDIOL | 7,00 |
| | **Allantoin** | ALLANTOIN | 0,20 |
| | | | |
| B | Olivem^{®} 1000 MB | CETEARYL OLIVATE, SORBITAN OLIVATE | 3,00 |
| | Lanette^{®} O | CETEARYL ALCOHOL | 2,00 |
| | Palmera A9818 | STEARIC ACID | 1,00 |
| | Myritol^{®} 318 | CAPRYLIC/CAPRIC TRIGLYCERIDE | 12,00 |
| | Cetiol^{®} C5 | COCO-CAPRYLATE | 5,00 |
| | LexFeel N200 | DIHEPTYL SUCCINATE, CAPRYLOYL GLYCERIN/SEBACIC ACID COPOLYMER | 2,00 |
| | **Mixed Tocopherols 95** | TOCOPHEROL | 0,15 |
| | | | |
| C | **STIMU-TEX^{®} AS** | ARGANIA SPINOSA KERNEL OIL, TOCOPHEROL, BUTYROSPERMUM PARKII BUTTER EXTRACT, SPENT GRAIN WAX | 3,00 |
| | **VALVANCE^{®} *Touch* 210** | SILICA | 1,00 |
| | Sepinov EMT 10 | HYDROXYETHYL ACRYLATE/SODIUM ACRYLOYLDIMETHYL TAURATE COPOLYMER | 0,80 |
| | | | |
| D | **PENTAVITIN^{®}** | SACCHARIDE ISOMERATE, AQUA, CITRIC ACID, SODIUM CITRATE | 1,00 |
| | Perfume Sail Away 49454695 | PARFUM | 0,10 |
| | **ALPAFLOR^{®} SCUTELLARIA CB** | GLYCERIN, AQUA, CITRIC ACID, SCUTELLARIA ALPINA FLOWER/LEAF/STEM EXTRACT | 1,00 |
| | **D-Panthenol 75 L** | PANTHENOL, AQUA | 1,00 |
| | **Vitamin B12** | CYANOCOBALAMIN | 0,001 - 0,5 |
| | | | |
| E | Sodium Hydroxide 10% Solution | SODIUM HYDROXIDE, AQUA | q.s. |

**10. Hand sanitizer**

| **Phase** | **ingredients** | **INCI Name** | **% w / w** |
|---|---|---|---|
| A | Water Dem | AQUA | Ad 100 |
| | Carbopol Ultrez 21 | ACRYLATES/C10-30 ALKYL ACRYLATE CROSSPOLYMER | 0,80 |
| | | | |
| B | Ethanol abs. | ALCOHOL | 65,00 |
| | | | |
| C | AMP Ultra PC 2000 | AMINOMETHYL PROPANOL | 0,06 |
| D | **DL-alpha-Tocopheryl Acetate** | TOCOPHERYLACETATE | 0,10 |
| | Eumulgin SML 20 | POLYSORBATE 20 | 0,20 |
| | | | |
| E | **Niacinamide PC** | NIACINAMIDE | 1,00 |
| | **D-Panthenol 75 L** | PANTHENOL, AQUA | 1,00 |
| | **TILAMAR^{®} PDO with NØØVISTA^{™}** | PROPANEDIOL | 3,00 |
| | **HYA-ACT^{™} XS** | HYDROLYZED HYALURONIC ACID | 0,10 |
| | | | |
| F | Eumulgin SML 20 | POLYSORBATE 20 | 0,20 |
| | Perfume Young Forever RT3770 | PARFUM | 0,10 |
| | **Vitamin B12** | CYANOCOBALAMIN | 0,001 - 0,5 |
| | | | |
| G | AMP Ultra PC 2000 | AMINOMETHYL PROPANOL | q.s. |

## Claims

1. Non-therapeutic use of Vitamin B12 as selective antimicrobial agent against *S. aureus.*

2. The use according to claim 1, wherein the antimicrobial effect of Vitamin B12 is **characterized by** a reduction in the share of *S. aureus* in the presence of one or both of *S. epidermidis* and/ or *S. hominis* in a skin microbiome.

3. The use according to claim 1 or 2, wherein the antimicrobial effect is **characterized in** a reduction in the share of *S. aureus* of at least 75% with respect to *S. epidermidis* and/ or *at least 10%, with respect to* S. hominis.

4. The use according to anyone of the preceding claims, wherein the antimicrobial effect is **characterized in** a reduction in the share of S. *aureus* of at least 80% with respect to *S. epidermidis* and/ or *at least* 35%, *with respect to* S. hominis.

5. The use according to anyone of the preceding claims, wherein the antimicrobial effect is **characterized in** a reduction in the share of *S. aureus* of 100%.

6. The use according to anyone of the preceding claims for maintaining a healthy skin microbiome.

7. The use according to anyone of the preceding claims, Vitamin B12 is incorporated into a cosmetic or dermatological composition.

8. The use according to claim 7, wherein the amount of Vitamin B12 in the cosmetic or dermatological composition is selected in the range of from 0.001 to 0.1 wt.-%, preferably from 0.001 to 0.05 wt.-%, most preferably from 0.001 to 0.025 wt.-%, based on the total weight of the composition.

9. A topical composition comprising Vitamin B12 for use in maintaining the balance of a healthy skin microbiome comprising at least *S. aureus* and one or both of *S. epidermidis* and *S. hominis.*

10. Vitamin B12 for the use in the prevention and/ or treatment of skin dysbiosis in a subject, wherein the skin dysbiosis is associated with an over-colonization by *S. aureus.*

11. A topical composition comprising Vitamin B12 for the use in the prevention or treatment of a disease associated with an over-colonization by *S. aureus.*

12. Vitamin B12 for use in counteracting the weakening of the skin barrier caused by a disbalance of the skin microbiome as well as in preventing or reducing the penetration of exogeneous molecules and/or microorganisms following such a weakening.

13. Vitamin B12 for use according to claim 12, wherein the disbalanced is caused by an over-colonization of the skin with *S. aureus.*

14. A method of suppressing the growth of *S. aureus* in a skin microbiome, the method comprising the step of topically administering a cosmetic or dermatological composition comprising Vitamin B12 to the skin, wherein the level of *S. aureus* in the skin microbiome of the human is lower relative to the level of *S. aureus* in the skin microbiome of a human administered a dermatological or cosmetic composition lacking the Vitamin B12.
